# EUROPEAN PATENT APPLICATION

(11) **EP 4 597 503 A1**
(43) Date of publication of application: **06.08.2025**
(21) Application number: 24305168.7
(22) Date of filing: 31.01.2024
(51) Int. Cl.: G16B 20/20, G16B 30/10

(54) **DEVICE AND METHOD FOR AUTOMATIC IDENTIFICATION OF REARRANGEMENTS IN GENOMIC REGIONS**

(71) Applicant: Seqone, 34000 Montpellier (FR)
(72) Inventor: BERTRAND, Denis, 34000 Montpellier (FR); MILLE, Marie, 34000 Montpellier (FR)
(74) Representative: Icosa

(57) **Abstract**

The present invention relates to devices and methods for automatic identification of rearrangements in genomic regions, and in particular, to devices and methods able to identify rearrangements in genomic regions with high sequence homology. The present invention also relates to methods for diagnosing diseases, and methods of treatment of diseases, comprising a step of automatic identification of rearrangements in genomic regions.

## Description

### FIELD OF INVENTION

The present invention relates to devices and methods for automatic identification of rearrangements in genomic regions, and in particular, to devices and methods able to identify rearrangements in genomic regions with high sequence homology. The present invention also relates to methods for diagnosing diseases, and methods of treatment of diseases, comprising a step of automatic identification of rearrangements in genomic regions.

### BACKGROUND OF INVENTION

Molecular diagnosis of patients presenting genetic abnormalities in genomic regions with high degree of sequence homology, such as, for example, the CFH gene cluster, is considered to be a challenging problem. In particular, those high repetitive genomic regions make the detection of genetic variations and particularly genetic rearrangement more difficult than in non-homologous regions. Some methods are described in the art as able to detect gene fusions using transcriptome sequencing. However, only manual methods exist for DNA sequencing, which raises concerns about the accuracy and feasibility of such manual methods. Thus, there is an urgent need to develop automatic and reliable methods enable to detect genetic rearrangements in regions with high degree of sequence homology.

Structural variations (SVs) and point mutations in the CFH gene cluster are associated with several complex genetic diseases, including age-related macular degeneration (AMD), atypical hemolytic uremic syndrome (AHUS), membranoproliferative glomerulonephritis (MPGN). Recently, SVs in the CFH cluster regions have also been reported to be associated with Complement-mediated thrombotic microangiopathy (cTMA). Importantly, the precise detection of those abnormalities is crucial for patient stratification and treatment decisions, leading to the need for an accurate methodology.

Herein, the Inventors have developed an automatic method that is able to accurately detect SVs in gene clusters, starting from long read sequencing data, and provide an application to the *CFH-CFHR* region in cTMA patients.

### SUMMARY

The present invention relates to a computer-implemented method for identification of a rearrangement between at least two genomic regions (x) and (x') in a subject; said method comprising:
- receiving reads from the sequencing of the genome of the subject, said reads being long-reads;
- mapping the reads on a reference genome;
- extracting the reads mapped on the first region (x) and/or on the second region (x');
- re-mapping the extracted reads on either the first region (x) or the second region (x');
- detecting single nucleotide variations (SNVs) on each read and associating each SNV with the region (x) or (x'), based on a predefined list of SNVs present in the genomic regions (x) and (x') of a reference genome;
- identifying a change of region (change from (x) to (x') or change from (x') to (x)), determining a breakpoint position in each read, and deducing the type of rearrangement between the at least two genomic regions (x) and (x') based on the change of region (change from (x) to (x') or change from (x') to (x)) and on the breakpoint position.

In one embodiment, the at least two genomic regions (x) and (x') present at least 70%, 75%, 80%, 85%, 95%, 96%, 97%, 98%, or at least 99% of sequence homology.

In one embodiment, the predefined list of SNVs is obtained by comparing the two regions (x) and (x') of a reference genome.

In one embodiment, the step of identifying a change of region, determining a breakpoint position in each read and deducing the type of rearrangement comprises a step of processing the data using sliding window approaches, statistical methods, and/or artificial intelligence-based methods.

In one embodiment, the long-reads are obtained from nanopore sequencing or HiFi sequencing.

In one embodiment, the genomic regions (x) and (x') are located in the complement factor H (CFH) gene cluster, wherein said CFH gene cluster comprises *CFH, CFHR1, CFHR2, CFHR3, CFHR4* and *CFHR5.*

In one embodiment, the genomic regions (x) and (x') are located in the *CFH-CFHR1* region.

In one embodiment, said first region (x) and said second region (x') have the following genomic coordinates:
- the region (x) has the genomic coordinates chr1:196711706-196740355 and the second region (x') has the genomic coordinates chr1:196796321-196825046, by reference to the human reference genome GRCh37, or
- the region (x) has the genomic coordinates chrl: 196742575-196771224 and the second region (x') has the genomic coordinates chrl: 196827190-196855915, by reference to the human reference genome GRCh38.

In one embodiment, the rearrangement in the CFH gene cluster is a *CFH-CFHR1* gene fusion or a *CFHRI-CFH* gene fusion, and/or any other rearrangements.

In one embodiment, the rearrangement in the CFH gene cluster is a *CFHR3-CFHR4* gene fusion.

In one embodiment, said method further comprises determining whether the rearrangement in the CFH gene cluster is monoallelic or biallelic.

In one embodiment, the subject suffers from or is diagnosed with a nephropathy.

In one embodiment, the subject suffers from or is diagnosed with a microangiopathy, preferably the subject suffers from or is diagnosed with a thrombotic microangiopathy (TMA), more preferably the subject suffers from or is diagnosed with a complement-mediated thrombotic microangiopathy (cTMA).

The present invention also relates to a device for identification of a rearrangement between at least two genomic regions (x) and (x') in a subject; said method comprising:
- at least one input configured to receive reads from the sequencing of the genome of the subject, said reads being long-reads;
- at least one processor configured to:
   - mapping the reads on a reference genome;
   - extracting the reads mapped on the first region (x) and/or on the second region (x');
   - re-mapping the extracted reads on either the first region (x) or the second region (x');
   - detecting single nucleotide variations (SNVs) on each read and associating each SNV with the region (x) or (x'), based on a predefined list of SNVs present in the genomic regions (x) and (x') of a reference genome;
   - identifying a change of region (change from (x) to (x') or change from (x') to (x)) and determining a breakpoint position in each read;
   - identifying a rearrangement between the at least two genomic regions (x) and (x') based on the change of region (change from (x) to (x') or change from (x') to (x)) and on the breakpoint position;

- at least one output configured to provide information on said identified rearrangement between the at least two genomic regions (x) and (x') based on the change of region (change from (x) to (x') or change from (x') to (x)) and said breakpoint position.

In addition, the disclosure relates to a computer program comprising software code adapted to perform a method for identification of a rearrangement between at least two genomic regions (x) and (x') in a subject, compliant with any of the above execution modes when the program is executed by a processor.

The present disclosure further pertains to a non-transitory program storage device, readable by a computer, tangibly embodying a program of instructions executable by the computer to perform a method for identification of a rearrangement between at least two genomic regions (x) and (x') in a subject, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

### DEFINITIONS

In the present invention, the following terms have the following meanings:
"**About**" preceding a figure encompasses plus or minus 10%, or less, of the value of said figure. It is to be understood that the value to which the term "about" refers is itself also specifically, and preferably, disclosed.

"**Complement factor H gene cluster**" or "C**FH gene cluster**": refers to a gene family cluster localized on chromosome 1q31.3 in humans. The CFH gene cluster comprises the six genes *CFH, CFHR3, CFHR1, CFHR4, CFHR2,* and *CFHR5.*

"**Genomic rearrangement**": refers to mutational changes in the genome such as deletion, insertion or inversion of DNA fragments on a genome.

"**Homology**" or "ide**n**tity": refers to the subunit sequence identity between two polymeric molecules, e.g., between two nucleic acid molecules, such as, two DNA molecules or two RNA molecules, or between two polypeptide molecules. When a subunit position in both of the two molecules is occupied by the same monomeric subunit; e.g., if a position in each of two DNA molecules is occupied by adenine, then they are homologous or identical at that position. The homology between two sequences is a direct function of the number of matching or homologous positions; e.g., if half (e.g., five positions in a polymer of ten subunits in length) of the positions in two sequences are homologous, the two sequences are 50% homologous; if 90% of the positions (e.g., 9 of 10), are matched or homologous, the two sequences are 90% homologous. Thus, the term "homologous" or "identical", when used in a relationship between the sequences of two or more polypeptides or of two or more nucleic acid molecules, refers to the degree of sequence relatedness between polypeptides or nucleic acid molecules, as determined by the number of matches between strings of two or more amino acid or nucleotide residues. "Identity" measures the percent of identical matches between the smaller of two or more sequences with gap alignments (if any) addressed by a particular mathematical model or computer program (i.e., "algorithms"). Identity of related polypeptides can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988). Preferred methods for determining identity are designed to give the largest match between the sequences tested. Methods of determining identity are described in publicly available computer programs. Preferred computer program methods for determining identity between two sequences include the GCG program package, including GAP (Devereux et al., Nucl. Acid. Res. \2, 387 (1984); Genetics Computer Group, University of Wisconsin, Madison, Wis.), BLASTP, BLASTN, and FASTA (Altschul et al., J. MoI. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity. Other methods include, for example, MUMmer (NUCmer). NUCmer pre-processes the DNA multi-FASTA input files so that they can be examined by the match finding routine. After which, the matches are clustered and the matches within clusters are extended via Smith-Waterman techniques in order to expand the total alignment coverage and close the gaps between clustered MUMs.

"**Long-reads**": refers to contiguous reads of at least 1000 bases.

"**Reference genome**": refers to a representative nucleic acid sequence database of a target species, obtained by assembling the DNA sequences from a number of individual subjects.

"**Single-nucleotide variations**" or "**SNV**": refers to a genomic variant at a single base position in the DNA.

"**Subject**", as used herein, refers to a mammal, preferably a human. In one embodiment, a subject may be a "**patient**", *i.e.,* a warm-blooded animal, more preferably a human, who/which is awaiting the receipt of, or is receiving medical care or was/is/will be the object of a medical procedure, or is monitored for the development of a disease.

"**Thrombotic microangiopathy**" or "**TMA**": refers to a pathology that results in thrombosis in capillaries and arterioles, due to an endothelial injury.

"**Treating**" or "**treatment**" or "**alleviation**" refers to both therapeutic treatment and prophylactic or preventative measures; wherein the object is to prevent or slow down (lessen) the targeted pathologic condition or disorder. Those in need of treatment include those already with the disorder as well as those prone to have the disorder or those in whom the disorder is to be prevented. In one embodiment, a subject is successfully "treated" for a disease, such as age-related macular degeneration (AMD) or a nephropathy, if, after receiving a therapeutic amount of the treatment according to the present invention, the subject shows at least one of the following: in the case of AMD, amelioration of vision and in the case of a nephropathy, amelioration of the kidney function. The above parameters for assessing successful treatment and improvement in the disease are readily measurable by routine procedures familiar to a physician.

The term "**processor**" should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

### BRIEF DESCRIPTION OF THE DRAWINGS

The present disclosure will be better understood, and other specific features and advantages will emerge upon reading the following description of particular and non-restrictive illustrative embodiments, the description making reference to the annexed drawings wherein:
**Figure 1** is a block diagram representing schematically a particular mode of a system for identifying rearrangements in genomic regions of a subject, compliant with the present disclosure.
**Figure 2** is a flow chart showing successive steps executed with the device for identification of figure 1.
**Figure 3** diagrammatically shows an apparatus integrating the functions of the device for identifying rearrangements in genomic regions of a subject of figure 1.
**Figure 4** is a schema showing the regions B and B' of high-homology, which are used to detect long-range structural variation in the *CFH* region.
**Figure 5** is a combination of one table and one graph referring to the analysis of sample 73. **Figure 5A****:** Automated method results table. **Figure 5B****:** Read SNV heatmap. A B/B' hybrid read, pointed by the arrow, is visible. Breakpoint region is indicated by a gray rectangle.
**Figure 6** is a combination of one table and one graph referring to the analysis of sample 75. **Figure 6A****:** Automated method results table. **Figure 6B****:** Read SNV heatmap. Three B/B' hybrid reads, pointed by the arrow, are visible. Breakpoint region is indicated by a gray rectangle.
**Figure 7** is a combination of one table and one graph referring to the analysis of sample 76. **Figure 7A****:** Automated method results table. **Figure 7B****:** Read SNV heatmap. Breakpoint region is indicated by a gray rectangle.
**Figure 8** is a combination of one table and one graph referring to the analysis of sample SYED. **Figure 8A****:** Automated method results table. **Figure 8B****:** Read SNV heatmap. The heatmap shows 7 B/B' hybrid reads are observed in the breakpoint region, and the absence of reads containing the B' region SNV from SNV number 1 to 259. Breakpoint region is indicated by a gray rectangle.

On the figures, the drawings are not to scale, and identical or similar elements are designated by the same references.

### DETAILED DESCRIPTION

All statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

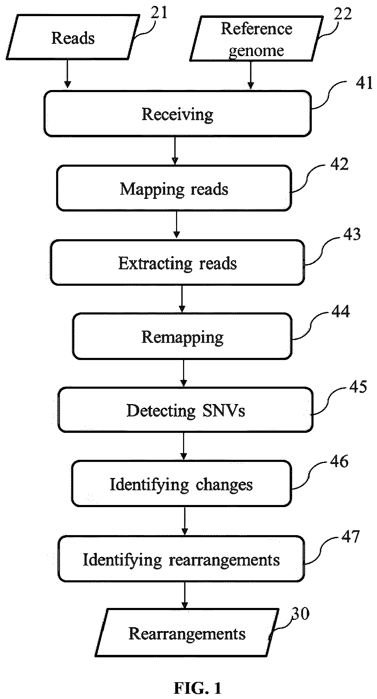

This invention relates to a computer-implemented method for identification of a rearrangement between at least two genomic regions (x) and (x') in a subject. As illustrated in Figure 1, said method comprises:
- receiving reads 21 from the sequencing of the genome of the subject and a reference genome 22, said reads being long-reads (step 41);
- mapping the reads on said reference genome 22 (step 42);
- extracting the reads mapped on the first region (x) and/or on the second region (x') (step 43);
- re-mapping the extracted reads on either the first region (x) or the second region (x') (step 44);
- detecting single nucleotide variations (SNVs) on each read and associating each SNV with the region (x) or (x'), based on a predefined list of SNVs present in the genomic regions (x) and (x') of a reference genome (step 45);
- identifying a change of region (change from (x) to (x') or change from (x') to (x)), determining a breakpoint position in each read (step 46), and deducing the type of rearrangement 30 between the at least two genomic regions (x) and (x') based on the change of region (change from (x) to (x') or change from (x') to (x)) and on the breakpoint position (step 47).

The method according to the present invention may be particularly advantageous for identification of a rearrangement between at least two genomic regions (x) and (x') with high degree of sequence homology.

Thus, in one embodiment, the at least two genomic regions (x) and (x') present a high degree of sequence homology.

As used herein, a high degree of sequence homology is equivalent to a "high sequence homology" or a "high percentage of sequence homology". It refers to a sequence homology of at least 70% between the at least two genomic regions (x) and (x').

In one embodiment, the at least two genomic regions (x) and (x') present at least 70%, 75%, 80%, 85%, 95%, 96%, 97%, 98%, or at least 99% of sequence homology. In a specific embodiment, the at least two genomic regions (x) and (x') present at least 97% of sequence homology.

In one embodiment, the method according to the present invention may further comprise a step of identifying at least two regions (x) and (x') with high sequence homology by aligning a region of interest comprising the two regions (x) and (x') against itself.

In one embodiment, the method according to the present invention is for identification of a rearrangement between two genomic regions (x) and (x') in a subject. In one embodiment, the method according to the present invention is for identification of a rearrangement between more than two genomic regions, such as three, four or more genomic regions.

In one embodiment, the rearrangement is a fusion of DNA fragments, a deletion of DNA fragments, an inversion of DNA fragments and/or any other rearrangement.

In one embodiment, the rearrangement is a fusion of genes, a deletion of genes an inversion of genes and/or any other rearrangement.

As disclosed herein, the method according to the present invention comprises a step of receiving reads from the sequencing of the genome of the subject, said reads being long-reads.

In one embodiment, the reads from the sequencing of the genome of the subject are obtained from a biological sample of the subject.

Examples of biological samples include, without limitation, blood sample or plasma sample.

In one embodiment, the reads are obtained from nanopore sequencing. As used herein, nanopore sequencing refers to a technology that enables direct, real-time analysis of long DNA or RNA fragments, relying on the use of protein nanopores.

In one embodiment, the reads are obtained from HiFi sequencing. As used herein, HiFi refers to high-fidelity long-read sequencing (Eid, J., et al. (2009) Real-time DNA sequencing from single polymerase molecules. Science, 323(5910), 133-138).

As disclosed herein, the method according to the present invention comprises a step of mapping (*i.e*. aligning) the reads on a reference genome and a step of extracting or identifying or selecting the reads mapped on the first region (x) and/or on the second region(x'). These steps enable to extract or identify or select the reads that align with the regions of interest (x) and/or (x').

Said reference genome may be obtained from databases. Examples of human reference genome are GRCh37 or GRCh38, released by the Genome Reference Consortium.

As disclosed herein, the method according to the present invention comprises a step of re-mapping the extracted reads on either the first region (x) or the second region (x'), and a step of detecting SNVs on each read and associating each SNV with the region (x) or (x'). These steps enable to detect each SNV present in each read from the subject and to determine whether each SNV originates from the region (x) or the region (x') (or an unknown region if the SNV is not associated with the regions (x) or (x')).

In one embodiment, the detection of SNVs and their association with the region (x) or (x') is based on the previous determination of a list of SNVs (*i.e.* a predefined list of SNVs) present in the genomic regions (x) and (x') of a reference genome.

In one embodiment, the predefined list of SNVs is obtained by comparing the two regions (x) and (x') of a reference genome. In this embodiment, each variation between the two regions is designated as an SNV, and is associated with either the region (x) or the region (x').

Thus, in one embodiment, the method according to the present invention may further comprise a step of defining a list of SNVs present in the genomic regions (x) and (x') of a reference genome.

In one embodiment, the method according to the present invention may further comprise a step of comparing the two regions (x) and (x') of a reference genome to define a list of SNVs present in the genomic regions (x) and (x') of a reference genome.

As disclosed herein, the method according to the present invention comprises a step of identifying a change of region (change from (x) to (x') or change from (x') to (x)) and determining a breakpoint position (*i.e.* a location in the genome where DNA might be deleted, inverted or duplicated).

As disclosed herein, the method according to the present invention comprises a step of deducing the type of rearrangement between the at least two genomic regions (x) and (x') based on the change of region (change from (x) to (x') or change from (x') to (x)) and on the breakpoint position.

In one embodiment, the steps of identifying a change of region, determining a breakpoint position and deducing the type of rearrangement are performed concomitantly.

It will be understood that, depending on the reads, one or several breakpoint position(s) may be found within one read. In case of several breakpoint positions within one read, the method according to the present invention comprises a step of determining each breakpoint position within a read.

In one embodiment, the step of identifying a change of region, determining a breakpoint position in each read and deducing the type of rearrangement comprises a step of processing the data to reduce the noise (*i.e.* a step of smoothing the data).

In one embodiment, the step of identifying a change of region, determining a breakpoint position in each read and deducing the type of rearrangement comprises a step of processing the data using sliding window approaches, statistical methods, and/or artificial intelligence-based methods.

In one embodiment, the step of identifying a change of region, determining a breakpoint position in each read and deducing the type of rearrangement comprises a step of processing the data using a sliding window approach.

In one embodiment, the sliding window approach comprises the following steps:
- using a sliding window comprising several SNVs to determine the region (*i.e.* the region (x) or (x')) for that window based on a majority rule,
- determining breakpoints positions based on change of the region (change from (x) to (x') or change from (x') to (x)),
- for each breakpoint, determining genomic coordinates and event type (*e.g,* deletion, hybrid CFH/CHR1).

In one embodiment, event types are determined depending on the change of region (change from (x) to (x') or change from (x') to (x)) and the genomic coordinates of the breakpoint in the regions (x) and (x').

In one embodiment, the step of identifying a change of region, determining a breakpoint position in each read and deducing the type of rearrangement comprises a step of discarding poor quality events, which advantageously allows to improve the sensitivity of the present method for identification. In one example, said step of discarding poor quality events comprises:
- computing a ratio of the number of SNVs linked to regions B or B' over the number of SNVs found on each side of the breakpoint (limited by another breakpoint or read boundaries), and
- comparing said ratios with a predefined reference threshold,
- discarding the events linked to the breakpoint if one of the ratios is below the predefined reference threshold.

In one embodiment, the predefined reference threshold may have a value comprising from about 0.60 to about 0.80, such as about 0.70.

In one embodiment, the step of identifying a change of region, determining a breakpoint position in each read and deducing the type of rearrangement comprises a step of processing the data using statistical methods, such as, for example, exponential smoothing or Kernel smoother.

In one embodiment, the step of identifying a change of region, determining a breakpoint position in each read and deducing the type of rearrangement comprises a step of processing the data using artificial intelligence-based methods.

In one embodiment, the step of identifying a change of region, determining a breakpoint position in each read and deducing the type of rearrangement comprises a step of clustering reads that share the same events.

In one embodiment, the clustering of reads that share the same event is performed by the following steps:
- Computing the occurrence of each event (*e.g*, deletion, hybrid CFH/CHR1) based on exact match of change of regions and breakpoint location
- creating a list of event each having : a change of region, a breakpoint location, a rearrangement type and a number of occurrence
- identifying the reads that have similar changes of region, similar breakpoint positions and determining the breakpoint position of reference, the breakpoint position of reference being the breakpoint position the most frequent in the reads,
- grouping reads that have similar changes of region, similar rearrangement and a breakpoint position that varies of 3 SNVs position at most before and after the breakpoint position of reference,
- once merged, events are discarded from the events to be merged and another run of merging is performed.

In one embodiment, the method according to the present invention is applied to the complement factor H (CFH) gene cluster. Thus, in one embodiment, the genomic regions (x) and (x') are located in the complement factor H (CFH) gene cluster, wherein said CFH gene cluster comprises *CFH, CFHR1, CFHR2, CFHR3, CFHR4* and *CFHR5.*

In one embodiment, the method according to the present invention enables to detect a rearrangement in the complement factor H (CFH) gene cluster.

In one embodiment, the method according to the present invention enables to determine whether the rearrangement in the CFH gene cluster is monoallelic or biallelic.

In one embodiment, the genomic regions (x) and (x') are located in the *CFH-CFHR1* region.

In one embodiment, the region (x) is a region having genomic coordinates chr1:196711706-196740355 and the region (x') is a region having the genomic coordinates chr1:196796321-196825046, by reference to the human reference genome GRCh37.

In one embodiment, the region (x) is a region having genomic coordinates chrl: 196742575-196771224 and the region (x') is a region having the genomic coordinates chrl: 196827190-196855915, by reference to the human reference genome GRCh38.

It will be understood that, based on the genomic coordinates described hereinabove, the skilled artisan in the art would be able to find the corresponding genomic coordinates in any other human reference genome.

In one embodiment, the rearrangement in the CFH gene cluster is a *CFH-CFHR1* gene fusion, a *CFHRI-CFH* gene fusion and/or any other rearrangement.

In one embodiment, the region (x) is a region covering partially *CFHR3* gene towards the end and the region (x') is a region covering partially *CFHR4* gene towards the end.

In one embodiment, the rearrangement in the CFH gene cluster is a *CFHR3-CFHR4* gene fusion.

As disclosed herein, the method according to the present invention enables to identify genomic rearrangements in a subject.

In one embodiment, the subject is a male. In one embodiment, the subject is a female. In one embodiment, the subject is an adult, *i.e.* with an age equal or above 18 years. In one embodiment, the subject is a child, *i.e.* with an age below 18 years.

In one embodiment, the subject suffers from or is diagnosed with a disease caused by rearrangements between at least two genomic regions (x) and (x').

In one embodiment, the disease is associated with rearrangements in the CFH gene cluster.

In one embodiment, the disease is age-related macular degeneration.

In one embodiment, the disease is a nephropathy.

In one embodiment, the disease is a microangiopathy. In one embodiment, the disease is a thrombotic microangiopathy (TMA). In one embodiment, the disease is a complement-mediated thrombotic microangiopathy (cTMA) (also known as an atypical hemolytic uremic syndrome (aHUS).

Examples of symptoms of TMA include, without limitation, fatigue, dizziness, shortness of breath, bleeding abnormalities, confusion, sleepiness, seizures, high blood pressure, fever, decreased urine.

The method according to the present invention may be particularly advantageous for diagnosing or assisting diagnosing a disease in a subject, wherein said disease is caused by rearrangements between at least two genomic regions (x) and (x').

Thus, the present invention further relates to a method for diagnosing a disease caused by rearrangements between at least two genomic regions (x) and (x') in a subject, the method comprising:
- performing the computer-implemented method for identification of a rearrangement between at least two genomic regions (x) and (x') in the subject, as disclosed herein, and
- diagnosing the disease depending on the rearrangement identified in the previous step.

In one embodiment, the disease is as defined hereinabove.

In one embodiment, the disease is a microangiopathy, preferably a TMA, more preferably a cTMA.

Thus, the present invention further relates to a method for diagnosing a TMA, preferably a cTMA, in a subject, the method comprising:
- performing the computer-implemented method for identification of a rearrangement between at least two genomic regions (x) and (x'), as disclosed herein,
- diagnosing a TMA, preferably a cTMA, if the rearrangement identified in the previous step is a *CFH-CFHR1* gene fusion, a *CFHRI-CFH* gene fusion, or a *CFHR3-CFHR4* gene fusion.

In one embodiment, the rearrangement identified in the previous step is a *CFH-CFHR1* gene fusion or a *CFHRI-CFH* gene fusion.

In one embodiment, the method for diagnosing a TMA, preferably a cTMA, comprises:
- performing the computer-implemented method as disclosed herein for identification of a rearrangement in the *CFH-CFHR1* region, and in particular, between:
   ∘ the region (x) having the genomic coordinates chr1:196711706-196740355 and the region (x') having the genomic coordinates chr1:196796321-196825046, by reference to the human reference genome GRCh37, or
   ∘ the region (x) having the genomic coordinates chrl: 196742575-196771224 and the region (x') having the genomic coordinates chrl: 196827190-196855915, by reference to the human reference genome GRCh38;
- diagnosing a TMA, preferably a cTMA, if the rearrangement identified in the previous step is a *CFH-CFHR1* gene fusion or a *CFHRI-CFH* gene fusion.

The present invention further relates to a method for treating a subject suffering from a disease caused by rearrangements between at least two genomic regions (x) and (x'), the method comprising:
- performing a computer-implemented method for identification of a rearrangement between at least two genomic regions (x) and (x') in the subject, as disclosed herein,
- diagnosing the subject with a disease depending on the rearrangement identified in the previous step, and
- treating the subject.

In one embodiment, the disease is as defined hereinabove.

In one embodiment, the disease is a microangiopathy, preferably a TMA, more preferably a cTMA.

Thus, the present invention further relates to a method for treating a subject suffering from a TMA, preferably a cTMA, the method comprising:
- performing a computer-implemented method for identification of a rearrangement between at least two genomic regions (x) and (x') in the subject, as disclosed herein,
- diagnosing the subject with a TMA, preferably a cTMA, if the genomic rearrangement identified in the previous step is a *CFH-CFHR1* gene fusion, a *CFHRI-CFH* gene fusion, or a *CFHR3-CFHR4* gene fusion, and
- treating the subject.

In one embodiment, the genomic rearrangement identified in the previous step is a *CFH-CFHR1* gene fusion or a *CFHRI-CFH* gene fusion.

In one embodiment, the method for treating a subject suffering from a TMA, preferably a cTMA, comprises:
- performing the computer-implemented method as disclosed herein for identification of a rearrangement in the *CFH-CFHR1* region, and in particular, between:
   ∘ the region (x) having the genomic coordinates chr1:196711706-196740355 and the region (x') having the genomic coordinates chr1:196796321-196825046, by reference to the human reference genome GRCh37, or
   ∘ the region (x) having the genomic coordinates chrl: 196742575-196771224 and the region (x') having the genomic coordinates chrl: 196827190-196855915, by reference to the human reference genome GRCh38;
- diagnosing the subject with a TMA, preferably a cTMA if the rearrangement identified in the previous step is a *CFH-CFHR1* gene fusion or a *CFHRI-CFH* gene fusion, and
- treating the subject.

Examples of treatment for cTMA include, for example, plasma infusion, plasma exchange, eculizumab and ravulizumab.

The disclosure also relates to a computer program comprising software code adapted to perform a method for identification of a rearrangement between at least two genomic regions (x) and (x') in a subject, compliant with any of the above execution modes when the program is executed by a processor.

The present disclosure further pertains to a non-transitory program storage device, readable by a computer, tangibly embodying a program of instructions executable by the computer to perform a method for identification of a rearrangement between at least two genomic regions (x) and (x') in a subject, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

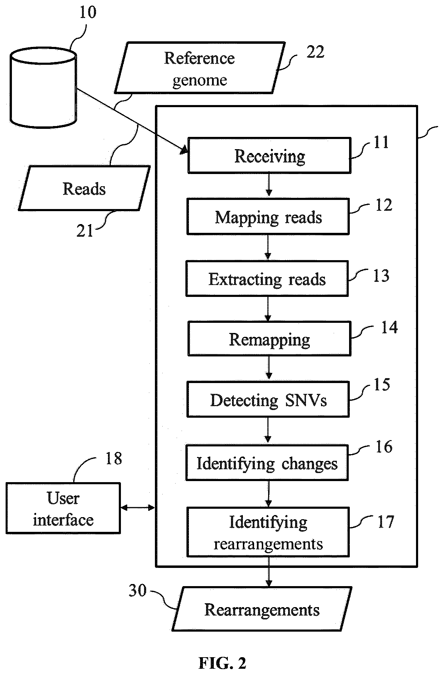

The present disclosure will be described in reference to a particular functional embodiment of a device 1 identification of a rearrangement between at least two genomic regions (x) and (x') in a subject, as illustrated on Figure 2.

Though the presently described device 1 versatile and provided with several functions that can be carried out alternatively or in any cumulative way, other implementations within the scope of the present disclosure include devices having only parts of the present functionalities.

Devices 1 is advantageously an apparatus, or a physical part of an apparatus, designed, configured and/or adapted for performing the mentioned functions and produce the mentioned effects or results. In alternative implementations, any of the device 1 is embodied as a set of apparatus or physical parts of apparatus, whether grouped in a same machine or in different, possibly remote, machines. The device 1 may e.g. have functions distributed over a cloud infrastructure and be available to users as a cloud-based service, or have remote functions accessible through an API.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor of the device 1.

The device 1 comprises a module 11 for implementing the step 41 of receiving the reads 21 and the reference genome 22, stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk).

The device 1 further comprises optionally a module for preprocessing the received reads 21. The module may notably be adapted to standardize the received reads 21 for sake of efficient and reliable processing.

The device 1 also comprises a module 12 for mapping reads according to step 42 of the method as described above.

The device 1 further comprises a module 13 for extracting the reads mapped on the first region (x) and/or on the second region (x') according to step 43 of the method as described above.

The device 1 comprises a module 14 for re-mapping the extracted reads on either the first region (x) or the second region (x') according to step 44 of the method as described above.

The device 1 comprises as well a module 15 for detecting single nucleotide variations (SNVs) on each read and associating each SNV with the region (x) or (x'), according to step 45 of the method as described above.

The device 1 comprises a module 16 for identifying a change of region (change from (x) to (x') or change from (x') to (x)) and determining a breakpoint position in each read according to step 46 of the method as described above.

The device 1 further comprises a module 17 for identifying a rearrangement 30 between the at least two genomic regions (x) and (x') based on the change of region (change from (x) to (x') or change from (x') to (x)) and on the breakpoint position according to step 47 of the method as described above.

The device 1 is interacting with a user interface 18, via which information can be entered and retrieved by a user. The user interface 18 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

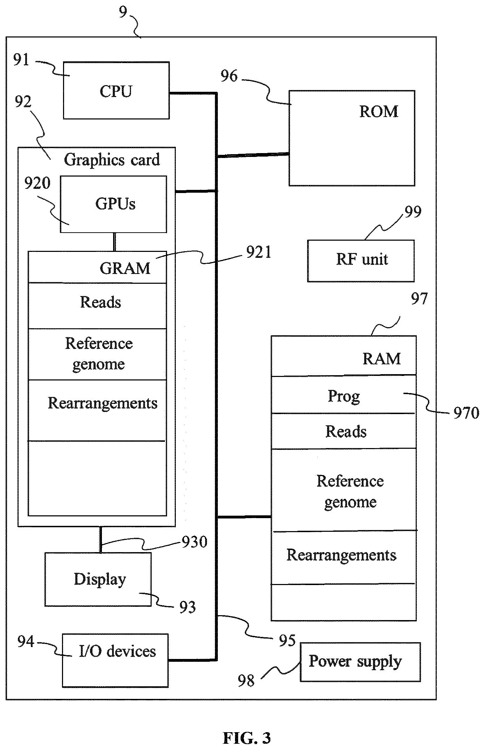

A particular apparatus 9, visible on Figure 3, is embodying the device 1 described above. It corresponds for example to a workstation, a laptop, a tablet, a smartphone, or a head-mounted display (HMD).

That apparatus 9 is suited to identification of a rearrangement between at least two genomic regions. It comprises the following elements, connected to each other by a bus 95 of addresses and data that also transports a clock signal:
- a microprocessor 91 (or CPU);
- a graphics card 92 comprising several Graphical Processing Units (or GPUs) 920 and a Graphical Random Access Memory (GRAM) 921; the GPUs are quite suited to image processing, due to their highly parallel structure;
- a non-volatile memory of ROM type 96;
- a RAM 97;
- one or several I/O (Input/Output) devices 94 such as for example a keyboard, a mouse, a trackball, a webcam; other modes for introduction of commands such as for example vocal recognition are also possible;
- a power source 98; and
- a radiofrequency unit 99.

According to a variant, the power supply 98 is external to the apparatus 9.

The apparatus 9 also comprises a display device 93 of display screen type directly connected to the graphics card 92 to display synthesized images calculated and composed in the graphics card. The use of a dedicated bus to connect the display device 93 to the graphics card 92 offers the advantage of having much greater data transmission bitrates and thus reducing the latency time for the displaying of information composed by the graphics card. According to a variant, a display device is external to apparatus 9 and is connected thereto by a cable or wirelessly for transmitting the display signals. The apparatus 9, for example through the graphics card 92, comprises an interface for transmission or connection adapted to transmit a display signal to an external display means such as for example an LCD or plasma screen or a video-projector. In this respect, the RF unit 99 can be used for wireless transmissions.

It is noted that the word "register" used hereinafter in the description of memories 97 and 921 can designate in each of the memories mentioned, a memory zone of low capacity (some binary data) as well as a memory zone of large capacity (enabling a whole program to be stored or all or part of the data representative of data calculated or to be displayed). Also, the registers represented for the RAM 97 and the GRAM 921 can be arranged and constituted in any manner, and each of them does not necessarily correspond to adjacent memory locations and can be distributed otherwise (which covers notably the situation in which one register includes several smaller registers).

When switched-on, the microprocessor 91 loads and executes the instructions of the program contained in the RAM 97.

As will be understood by a skilled person, the presence of the graphics card 92 is not mandatory, and can be replaced with entire CPU processing and/or simpler visualization implementations.

In variant modes, the apparatus 9 may include only the functionalities of the device 1. In addition, the device 1 may be implemented differently than a standalone software, and an apparatus or set of apparatus comprising only parts of the apparatus 9 may be exploited through an API call or via a cloud interface.

### EXAMPLES

The present invention is further illustrated by the following examples.

### Example 1:

### Materials and Methods

Our invention addresses a complex technical challenge: the detection of long-range rearrangements in the CFH genes cluster region. This invention is designed to identify specific genomic alterations, including but not limited to *CFH-CFHR1* gene fusions, *CFHRI-CFH* gene fusions, and deletions of the *CFHR3* and *CFHR1* genes. Additionally, it can detect any other rearrangements that occur within the B-B' regions (see below definitions of B and B').

### Detection of high-homology regions in the CFH genes cluster

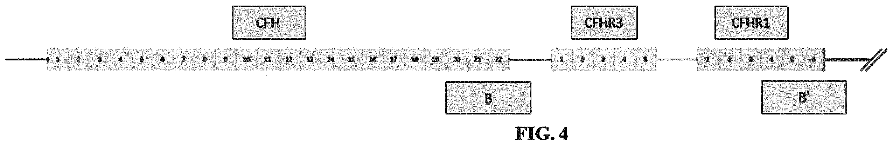

To detect the aforementioned alterations, the method is based on the analysis of two genomic regions named B and B'. The regions B and B' are regions with high similarities. Their localization is described on Figure 4.

To identify these high-homology regions, we performed a self-alignment of the *CFH* cluster area. This was accomplished using nucmer, a tool within the mummer tools suite. The parameters selected for nucmer were '--maxmatch', which utilizes all maximal exact matches as alignment anchors, and '--nosimplify' which is a specific option when aligning a sequence to itself. This process led us to discover two regions with a homology of 97.3% that corresponds to a known recombination hotspot between the genes *CFH* and *CFHR1.* These regions, located on chromosome 1, are defined by the following coordinates on reference genome GRCh38:
- Region B: 196742575-196771224
- Region B': 196827190-196855915

Performing an alignment between the region B and B' using nucmer (default parameters), we generated a list of single nucleotide variations (SNV) and their positions respectively on B and B 'is generated. Variants were called using 'show-snps' from the mummer tools suite, only SNV were retained to produce what is called the SNV list. This list determines the differences between the 2 regions.

### Detection of chimeric reads between B and B'

The invention is built on the principle that if there is a read that encompasses both region B and B', then there is a structural rearrangement in the *CFH* genes cluster region. To identify if a read belongs to region B or B', we look at the SNVs within the read. If the SNVs match those known to be in region B, then we can infer that the read came from region B. Similarly, if the SNVs match those in region B', then the read likely came from region B'. If a read contains stretches of SNVs from both regions, it could indicate that the read spans both regions.

The method to detect chimeric reads is described below. It is based on two input files consisting of the list of SNV between B and B' (SNV list) and of the mapping between the Nanopore long reads and the reference genome.

Firstly, all reads sequenced with Oxford Nanopore technology are mapped on reference genome using minimap2 with specific parameters for Oxford nanopore long reads (-ax map-ont). Then, reads mapped on region B and B' are extracted and mapped against region B.

Next, for each read, all SNV positions from the SNV_list are assigned to a region, by allocating them a value as follows: B = 0, B' = 1, or -1 for others variation or not covered position.

A sliding window of 20 consecutive SNV positions is used to set a region (B or B') for that window. To set a region for the window we will use a majority rule. If we have a majority of SNVs from region B, the region B will be affected to that window and same with region B'. By examining all windows, the change of region between two consecutive windows is detected.

Subsequently, identified windows with a change of region are processed to pinpoint a more precise breakpoint position. The designation of that breakpoint is achieved by observing the first change of region within that window. If another breakpoint exists within the same window, the change of region from the previous breakpoint becomes the starting point of the examination.

Once the position of the breakpoint and the region on each side of it have been determined, the alteration type for that breakpoint is identified. This could include alterations such as *CFH-CFHR1* gene fusions, *CFHRI-CFH* gene fusions, deletions of the *CFHR3 and CFHR1* genes, or other rearrangements occurring in B-B' regions.

In order to filter out low-quality events, the ratio of SNVs linked to regions B or B' to the number of SNVs found on each side of the breakpoint (limited by another breakpoint or read boundaries) is calculated. For instance, events linked to a breakpoint are retained if ratios on each side exceed 0.7. Concurrently, the number of occurrences of identical changes of region at the same breakpoint position is computed.

One of the final steps is the clustering of the events to generate a synthetic table. Events with the same type of alteration and with a close breakpoint position will be merged. A core event for a type of alteration is determined by the higher number of occurrences. All events of the same type within a range of 3 SNVs positions are combined.

To analyze results, a table with the events merged and the type of rearrangement is provided. Also, a heatmap representing each SNV from the SNV_list and their associated region for all reads is generated.

### Results

In the present study, we compared the performance of our novel method with the Multiplex Ligation-dependent Probe Amplification (MLPA) technique. MLPA analyses are considered as the truth. Three distinct samples were analyzed using both methods to ensure a comprehensive comparison.

We validated structural variation events on 3 samples using an MPLA assay targeting (in genomic order) *CFH* exons 1 to 22, *CFHR3* exons 1, 2, 3, 4, 6, *CFHR1* exons 3, 5, 6, *CFHR4* exons 1, 2, 4, 6, *CFHR2* exons 1 to 5 and *CFHR5* exons 1 to 3.

Our method demonstrated a high level of accuracy across all samples. We have a strong positive relationship between the predicted and actual values. This suggests that our method is capable of producing reliable and consistent results.

The heatmap provided represents a read for each line and column indicates the nth variation in the list (result table indicates the breakpoint position in this coordinate system) and each cell indicates the region associated to the SNV on a read. A gray cell corresponds to B region SNV, a white cell to a B' region SNV and a black cell to non-covered region or variation not matching B or B' region SNVs. Using this representation, B/B' chimeric reads can be identified as a stretch of gray cells followed by a stretch of white cells.

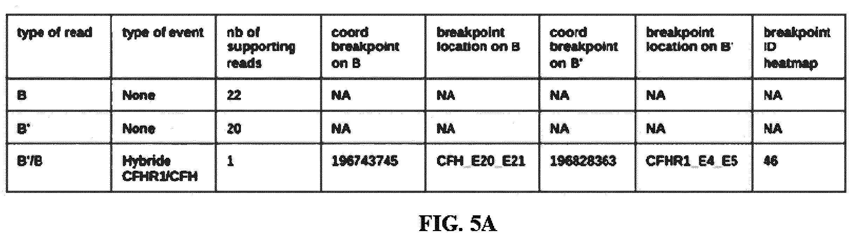

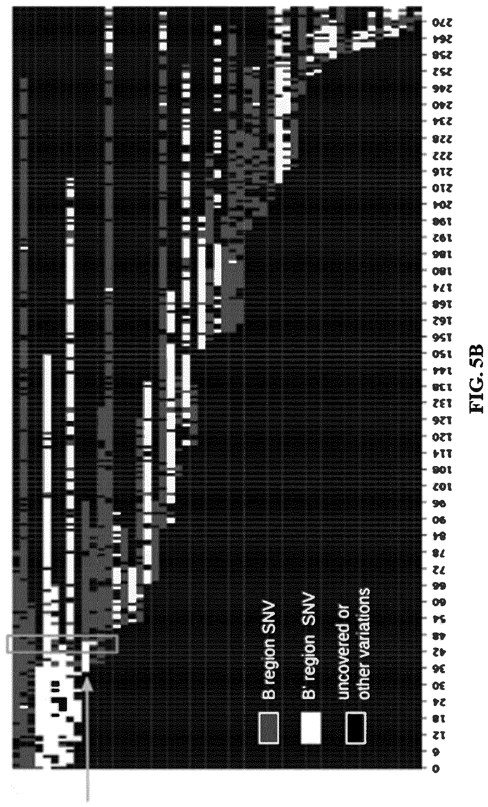

On the sample 73, the automated method detected a single chimeric B'/B read supporting an event of fusion *CFHR1* exon *4*/*CFH* exon 21 (Figures 5A and 5B). Notice that due to the low coverage of the sample, the breakpoint region is only covered by 6 interpretable reads: 3 from B, 2 from B' and 1 chimeric B'/B read. This corresponds to the expected read ratio as the sample genome contains 2 B regions, 2 B' regions and a single B/B' region in case of an heterozygous *CFHRI-CFH* fusion. The breakpoint location and the event identified is confirmed by the MLPA results where we observed a 50% increase of the copy of number in the *CFH* exon 20 to 22, all *CHFR3* exons and *CFHR1* exon 3 compared to *CFH* exons 1 to 19, *CFHR1* exons 5 to 6 and all *CFHR4, CFHR2,* and *CFHR5* exons.

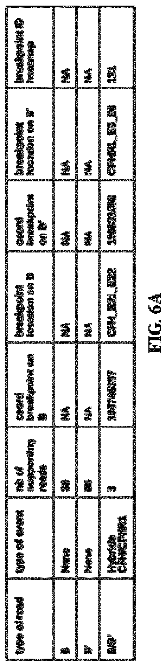

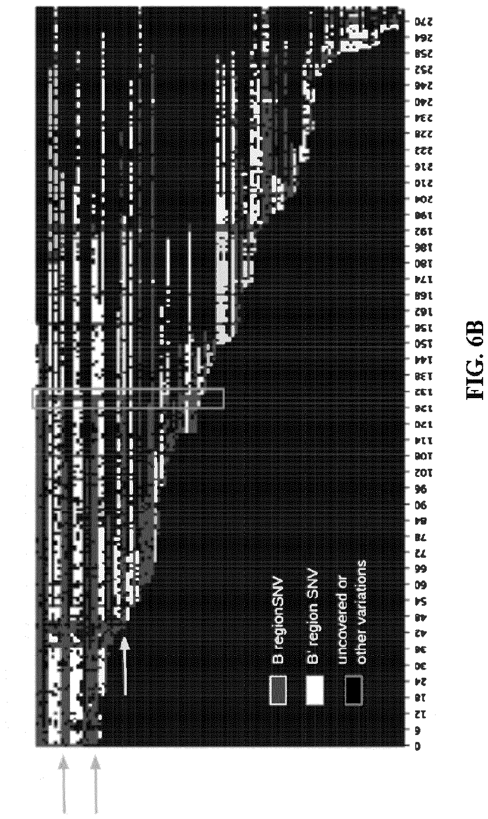

On the sample 75, the automated method detected 3 chimeric B/B' reads supporting an event of fusion *CFH* exon 21/*CFHR1* exon 6 (Figures 6A and 6B). The breakpoint location and the event identified are confirmed by the MLPA results where we observed a 50% depletion of the copy number of *CFH* exons 22, all *CFHR3* exons, *CHFR1* exon 3 and 5 compared to *CHF* exons 1 to 21, *CFHR1* exon 6 and all *CFHR4, CFHR2,* and *CFHR5* exons.

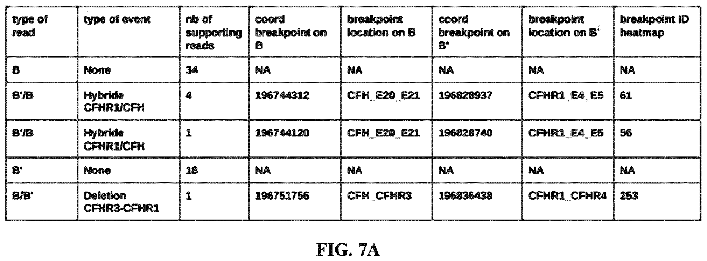

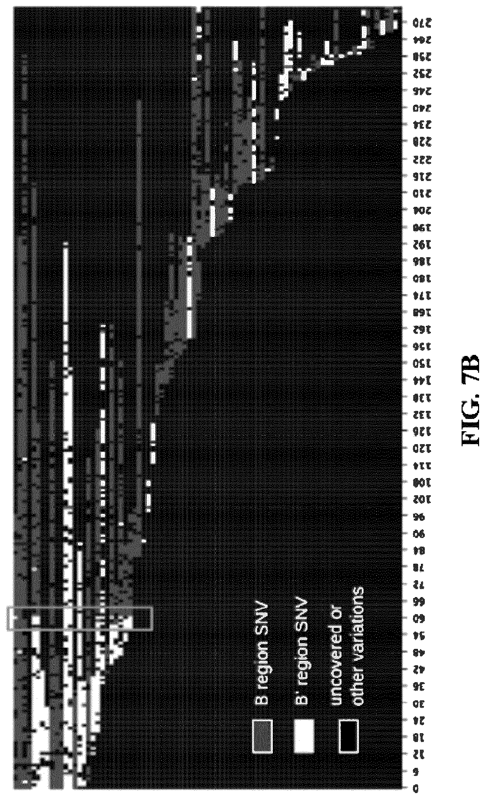

On the sample 76, the automated method detected 4 B'/B chimeric reads supporting an event of *CFHR1* exon *4*/*CFH* exon 21 fusion (Figures 7A and 7B). The breakpoint location and the event identified are confirmed by the MLPA results where we observed a 50% increase in copy number of CFH exons 21 and 22, and a 50% depletion *CFHR1* exons 5 and 6 compared to CFH exons 1 to 20, *CFHR1* exon 3 and all *CFHR2, CFHR4* and *CFHR5* exons. The automated method identified 2 other variants supported by a single read: another B/B' chimeric read less than 200bp away from the identified breakpoint that potentially indicates the same event, and, in a low coverage region, a *CFHR1*/*CFHR3* deletion. This deletion is not identified by the MLPA analysis and does not affect the patient diagnostic.

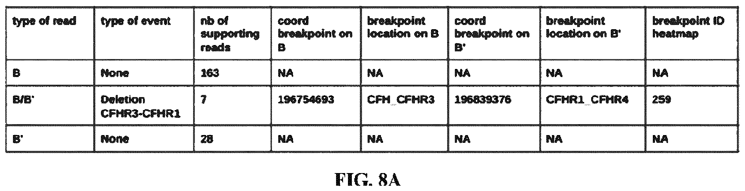

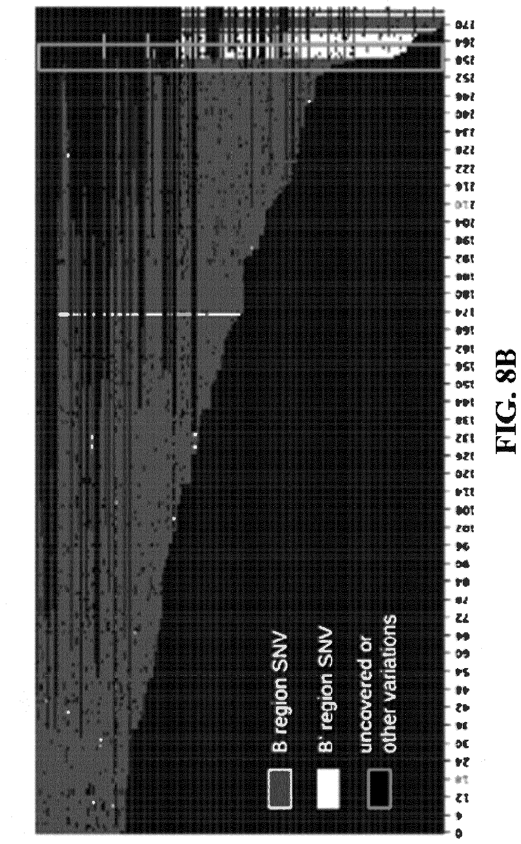

On the sample SYED, the automated method identified a homozygous *CFHR1-CFHR3* deletion event supported by 7 B/B' chimeric reads (Figures 8A and 8B). This event is further supported by the absence of reads containing the B' region SNV from SNV number 1 to 259. This event is confirmed by a short read coverage analysis where we observed a strong depletion of the coverage of *CFHRI-CFHR3* exons compared to the exons of the other genes of the cluster (*CFH, CFHR2, CFHR4,* and *CFHR5*)*.*

## Claims

1. A computer-implemented method for identification of a rearrangement between at least two genomic regions (x) and (x') in a subject; said method comprising:
- receiving reads from the sequencing of the genome of the subject, said reads being long-reads;
- mapping the reads on a reference genome;
- extracting the reads mapped on the first region (x) and/or on the second region (x');
- re-mapping the extracted reads on either the first region (x) or the second region (x');
- detecting single nucleotide variations (SNVs) on each read and associating each SNV with the region (x) or (x'), based on a predefined list of SNVs present in the genomic regions (x) and (x') of a reference genome;
- identifying a change of region (change from (x) to (x') or change from (x') to (x)), determining a breakpoint position in each read, and deducing the type of rearrangement between the at least two genomic regions (x) and (x') based on the change of region (change from (x) to (x') or change from (x') to (x)) and on the breakpoint position.

2. The method according to claim **1,** wherein the at least two genomic regions (x) and (x') present at least 70%, 75%, 80%, 85%, 95%, 96%, 97%, 98%, or at least 99% of sequence homology.

3. The method according to claim **1** or claim **2,** wherein the predefined list of SNVs is obtained by comparing the two regions (x) and (x') of a reference genome.

4. The method according to any one of claims **1** to **3,** wherein the step of identifying a change of region, determining a breakpoint position in each read and deducing the type of rearrangement comprises a step of processing the data using sliding window approaches, statistical methods, and/or artificial intelligence-based methods.

5. The method according to any one of claims **1** to **4,** wherein the long-reads are obtained from nanopore sequencing or HiFi sequencing.

6. The method according to any one of claims **1** to **5,** wherein the genomic regions (x) and (x') are located in the complement factor H (CFH) gene cluster, wherein said CFH gene cluster comprises *CFH, CFHR1, CFHR2, CFHR3, CFHR4* and *CFHR5.*

7. The method according to claim **6,** wherein said first region (x) and said second region (x') have the following genomic coordinates:
- the region (x) has the genomic coordinates chr1:196711706-196740355 and the second region (x') has the genomic coordinates chr1:196796321-196825046, by reference to the human reference genome GRCh37, or
- the region (x) has the genomic coordinates chrl: 196742575-196771224 and the second region (x') has the genomic coordinates chrl: 196827190-196855915, by reference to the human reference genome GRCh38.

8. The method according to claim **7,** wherein the rearrangement in the CFH gene cluster is a *CFH-CFHR1* gene fusion or a *CFHRI-CFH* gene fusion, and/or any other rearrangements.

9. The method according to claim **6,** wherein the rearrangement in the CFH gene cluster is a *CFHR3-CFHR4* gene fusion.

10. The method according to any one of claims **1** to **9,** wherein said method further comprises determining whether the rearrangement in the CFH gene cluster is monoallelic or biallelic.

11. The method according to any one of claims **1** to **10,** wherein the subject suffers from or is diagnosed with a nephropathy.

12. The method according to any one of claims **1** to **11,** wherein the subject suffers from or is diagnosed with a microangiopathy, preferably the subject suffers from or is diagnosed with a thrombotic microangiopathy (TMA), more preferably the subject suffers from or is diagnosed with a complement-mediated thrombotic microangiopathy (cTMA).

13. A device for identification of a rearrangement between at least two genomic regions (x) and (x') in a subject; said method comprising:
- at least one input configured to receive reads from the sequencing of the genome of the subject, said reads being long-reads;
- at least one processor configured to:
- mapping the reads on a reference genome;
- extracting the reads mapped on the first region (x) and/or on the second region (x');
- re-mapping the extracted reads on either the first region (x) or the second region (x');
- detecting single nucleotide variations (SNVs) on each read and associating each SNV with the region (x) or (x'), based on a predefined list of SNVs present in the genomic regions (x) and (x') of a reference genome;
- identifying a change of region (change from (x) to (x') or change from (x') to (x)) and determining a breakpoint position in each read;
- identifying a rearrangement between the at least two genomic regions (x) and (x') based on the change of region (change from (x) to (x') or change from (x') to (x)) and on the breakpoint position;
- at least one output configured to provide information on said identified rearrangement between the at least two genomic regions (x) and (x') based on the change of region (change from (x) to (x') or change from (x') to (x)) and said breakpoint position.
